# EUROPEAN PATENT APPLICATION

(11) **EP 4 379 358 A1**
(43) Date of publication of application: **05.06.2024**
(21) Application number: 22849688.1
(22) Date of filing: 20.05.2022
(51) Int. Cl.: G01N 21/64, G01N 21/25, G02B 6/28, G02B 6/02, G02B 6/32, A61B 5/00, A61N 5/067

(54) **MULTI-SPECTRUM LIGHT IRRADIATION APPARATUS AND MULTI-SPECTRUM FLUORESCENCE IMAGING SYSTEM**

(30) Priority: 28.07.2021 KR 20210098909
(71) Applicant: Metaplebio Co., Ltd., Seoul 06677 (KR)
(72) Inventor: HAN, Young Geun, Seoul 05834 (KR); PARK, Hong Seong, Seoul 02560 (KR)
(74) Representative: Sander, Rolf
(86) International application number: PCT/KR2022/007227
(87) International publication number: WO 2023/008709

(57) **Abstract**

Proposed are a multi-spectrum light-emitting apparatus and a multi-spectrum fluorescence imaging system. The multi-spectrum light-emitting apparatus includes a light source releasing light having a plurality of wavelengths, a light separator separating the light having the plurality of wavelengths into a plurality of individual beams of light having individual wavelengths, or into a plurality of individual beams of light having predetermined power levels, a plurality of multi-mode fibers, the plurality of individual beams of light, resulting from the separation by the light separator, being transferred over the plurality of multi-mode fibers, respectively, and light emitters connected to respective end portions of the plurality of multi-modal fibers, thereby reducing noise in the plurality of individual beams of light and emitting the resulting plurality of individual beams of light, along with illumination light, to a sample.

## Description

### [Technical Field]

The present disclosure relates to a multi-spectrum light-emitting apparatus and a multi-spectrum fluorescence imaging system, and more particularly, to a multi-spectrum light-emitting apparatus and a multi-spectrum fluorescence imaging system, the apparatus and the system being capable of measuring and identifying a plurality of fluorescent images resulting from a laser emitted in a plurality of wavelengths and reducing noise.

### [Background Art]

In a clinical trial, animal testing, image-guided surgery, and the like, a typical fluorescent imaging system may collect exciting light having one wavelength, may allow the exciting light to pass through, may emit the exciting light to a sample, that is, an observation-target body portion, and may observe a change in the sample or a structure of the sample, which results from an optical signal reflected off from the sample.

This use of the exciting light in a signal wavelength in the fluorescent imaging system makes it impossible to excite a luminous body using various wavelengths. In addition, it is impossible to vary a wavelength of the exciting light in a light source, thereby making it impossible to simultaneously observe multi-fluorescent images having a multi-spectrum.

In addition, when the exciting light having one wavelength is released from the light source, it is impossible to adjust a field-of-view area in a single light source or between a plurality of overlapping light sources. Furthermore, in a case where a laser light source is used, speckle noise due to interference phenomenon between scattering beans of light and multi-modal noise due to a multiplicity of modes may occur, thereby causing a decrease in the quality of an acquired fluorescent image.

The "speckle noise" here refers to noise that manifests as a stained grain-shaped pattern. This pattern results from glare and shadow being irregularly distributed due to a phenomenon of random interference between scattering beams of light. In a case where a laser is used as a light source, the scattering beams of light occur when the laser light source emits light to a sample or dye. That is, the speckle noise occurs when bright or dark dots are scattered on a captured image. This phenomenon occurs due to interference resulting from the laser scattering in various directions. In addition, the multi-modal noise refers to a multi-modal pattern noise that occurs due to a multiplicity of modes formed while the laser passes through a multi-modal optical fiber. The multi-modal noise may decrease the quality of the fluorescent image.

In a case where the laser is used as a light source in this manner, the quality of the captured fluorescent image decreases due to the speckle noise and the multi-modal noise, thereby decreasing the precision of image reading and requiring additional imaging. These problems lead to an increase in cost and time.

The above-mentioned matter in the background art is technical information that the inventor already retains to contrive the present disclosure, or technical information that is acquired by the inventor when contriving the present disclosure. Therefore, the above-mentioned matter cannot be necessarily regarded as a technology publicly known to the public before the present disclosure is filed with the Korean Intellectual Property Office.

(Patent Document ) Korean Patent Application Publication No. 10-2019-0067337 (June 17, 2019)

### [Disclosure]

### [Technical Problem]

One object of the present disclosure, which is contrived to address the above-mentioned problems, is to provide a multi-spectrum light-emitting apparatus and a multi-spectrum fluorescence imaging system, the apparatus and the system being capable of acquiring and distinguishing fluorescent images having different wavelengths, through a wavelength-variable light source, a multi-wavelength light source, a multi-array light source, or the like.

Another object of the present disclosure, which is contrived to address the above-mentioned problems, is to provide a multi-spectrum light-emitting apparatus and a multi-spectrum fluorescence imaging system, the apparatus and the system being capable of reducing speckle noise and multi-modal noise due to a laser using a noise reducer mounted on a light emitter.

Still another object of the present disclosure, which is contrived to address the above-mentioned problems, is to provide a multi-spectrum light-emitting apparatus and a multi-spectrum fluorescence imaging system, the apparatus and the system being capable of performing separation into a visible light signal and fluorescent signals having different wavelengths, using a light separator and of simultaneously outputting the visible light signal and the fluorescent signals.

Yet another object of the present disclosure, which is contrived to address the above-mentioned problems, is to provide a multi-spectrum light-emitting apparatus and a multi-spectrum fluorescence imaging system, the apparatus and the system being capable of detecting fluorescent signals having different wavelengths by releasing a laser in a range of specific wavelengths for necrosis of a specific cell or for treatment, over an optical fiber to which a noise reducer is not coupled and of simultaneously treating a diseased portion by emitting the laser thereto.

The present disclosure is not limited to the above-mentioned objects. An object not mentioned would be apparent from the following description to a person of ordinary skill in the art.

### [Technical Solution]

In order to accomplish the above-mentioned objects, according to one aspect of the present disclosure, there is provided a multi-spectrum light-emitting apparatus including: a light source releasing light having a plurality of wavelengths; a light separator separating the light having the plurality of wavelengths into a plurality of individual beams of light having individual wavelengths, or into a plurality of individual beams of light having predetermined power levels; a plurality of multi-mode fibers, the plurality of individual beams of light, resulting from the separation by the light separator, being transferred over the plurality of multi-mode fibers, respectively; and light emitters connected to respective end portions of the plurality of multi-mode fibers, thereby reducing noise in the plurality of individual beams of light and emitting the resulting plurality of individual beams of light, along with illumination light, to a sample.

In the multi-spectrum light-emitting apparatus, the light source may be a wavelength-variable light source, a multi-wavelength light source, or a multi-array light source, and may release light that varies in wavelength over time.

The multi-spectrum light-emitting apparatus may further include a light source multi-mode fiber connecting between the light source and the light separator.

In the multi-spectrum light-emitting apparatus, the light emitter may include: a plurality of noise reducers; a coupling disk configured in such a manner that the plurality of noise reducers are arranged to be spaced a predetermined distance apart from each other; and an illumination fixture coupled to one surface of the coupling disk and arranged to surround the vicinity of the plurality of noise reducers, and the illumination fixture may release the illumination light, and the illumination light may be white light.

In the multi-spectrum light-emitting apparatus, the number of the plurality of noise reducers may be smaller than the number of the plurality of multi-mode fibers, and the multi-mode fiber to which the noise reducer is not coupled may release a ray of light having a wavelength of between 200 nm to 450 nm, a ray of light having a wavelength of between 510 nm to 550 nm, a beam of near-infrared light having a wavelength of between 790 nm to 980 nm, or a beam of near-infrared light having a wavelength of between 1044 nm to 1084 nm.

In the multi-spectrum light-emitting apparatus, the plurality of noise reducers may be connected to the plurality of multi-mode fibers, respectively, thereby reducing speckle noise and multi-modal noise in the plurality of individual beams of light transferred over the plurality of multi-mode fibers, respectively.

In the multi-spectrum light-emitting apparatus, each of the plurality of noise reducers may include: a cylindrical housing; an optical fiber connection portion connected to the multi-mode fiber at one end portion of the cylindrical housing; a glass pane installed at the other end portion of the cylindrical housing; an aspherical lens arranged adjacent to the optical fiber connection portion within the cylindrical housing; and a homogenizing lens or diffusing lens arranged adjacent to the glass pane within the cylindrical housing.

In order to accomplish the above-mentioned object, according to another aspect of the present disclosure, there is provided a multi-spectrum fluorescence imaging system including: the multi-spectrum light-emitting apparatus above mentioned; a multi-spectrum light detection apparatus detecting a near-infrared fluorescent signal that is emitted from the multi-spectrum light-emitting apparatus to the sample and is released from the sample; and a control apparatus synchronizing the multi-spectrum light-emitting apparatus and the multi-spectrum light detection apparatus with each other.

In the multi-spectrum fluorescence imaging system, the multi-spectrum light detection apparatus may include: a wavelength separator separating an optical signal into a visible light signal reflected off from the sample and the near-infrared fluorescent signal released from the sample; a band rejection filter arranged between the sample and the wavelength separator; a near-infrared image sensor detecting the near-infrared fluorescent signal; and a visible light image sensor detecting the visible light signal.

The multi-spectrum fluorescence imaging system may further include a wheel filter arranged between the wavelength separator and the near-infrared image sensor or between the wavelength separator and the band rejection filter.

In the multi-spectrum fluorescence imaging system, the control apparatus may synchronize a time between the light source and the near-infrared image sensor and a wavelength of light, which corresponds to the time, may synchronize a time between the light source and the wheel filter and a wavelength of light, which corresponds to the time, and may synchronize a time between the wheel filter and the near-infrared image sensor and a wavelength of light, which corresponds to the time.

### [Advantageous Effects]

With a multi-spectrum light-emitting apparatus and a multi-spectrum fluorescence imaging system according to embodiments of the present disclosure, fluorescent images having different wavelengths can be acquired and distinguished.

In addition, with the multi-spectrum light-emitting apparatus and the multi-spectrum fluorescence imaging system according to the embodiments of the present disclosure, speckle noise and multi-modal noise can be reduced.

Furthermore, with the multi-spectrum light-emitting apparatus and the multi-spectrum fluorescence imaging system according to the embodiments of the present disclosure, separation into visible light signal and fluorescent signals having different wavelengths can be performed, and the visible light signal and the fluorescent signals can be simultaneously output.

Furthermore, with the multi-spectrum light-emitting apparatus and the multi-spectrum fluorescence imaging system according to the embodiments of the present disclosure, the fluorescent signals having different wavelengths can be detected, and a laser can be simultaneously emitted to a diseased portion for treatment.

The present disclosure is not limited to the above-mentioned effects. Effects not mentioned would be apparent from the following description to a person of ordinary skill in the art to which the present disclosure pertains.

### [Description of Drawings]

FIG. 1 is a view schematically illustrating a multi-spectrum fluorescence imaging system according to a first embodiment of the present disclosure.
FIG. 2 is a schematic block diagram illustrating an entire configuration of the multi-spectrum fluorescence imaging system according to the first embodiment of the present disclosure.
FIG. 3 is a schematic block diagram illustrating a multi-spectrum light-emitting apparatus according to the first embodiment of the present disclosure.
FIG. 4 is an exploded perspective view illustrating a light emitter according to the first embodiment of the present disclosure.
FIG. 5 is a cross-sectional view illustrating a noise reducer according to the first embodiment of the present disclosure.
FIG. 6 is an exploded perspective view illustrating a light emitter according to a second embodiment of the present disclosure.
FIG. 7 is a schematic block diagram illustrating a multi-spectrum light detection apparatus according to the first embodiment of the present disclosure.
FIG. 8 is a block diagram that is referenced to describe a method of synchronizing the multi-spectrum light-emitting apparatus and the multi-spectrum light detection apparatus with each other in the multi-spectrum fluorescence imaging system according to the first embodiment of the present disclosure.

### [Best Mode]

Advantages and features of the present disclosure, and methods of achieving the advantages and the features will be apparent from embodiments that will be described in detail below with reference to the accompanying drawings. However, the present disclosure is not limited to the embodiments that will be disclosed below and may be implemented in various different forms. The embodiments are only provided to make the present disclosure complete and to provide definite notice as to the scope of the present disclosure to a person of ordinary skill in the art to which the present disclosure pertains. The scope of the present disclosure should be only defined by claims.

Shapes, sizes, scales, angles, quantities, and the like that are illustrated in the drawings for description of the present disclosure are only given as examples and thus do not impose any limitation to the present disclosure. In addition, a specific description of a well-known technology associated with the present disclosure will be omitted when determined as making the nature and gist of the present disclosure obfuscated. The verbs "include," "have," "is configured with", and the like, which are used in the present specification, as long as the modifier "only" is not used, may allow for addition of one or more other components. Unless explicitly stated otherwise, a constituent element expressed in single form may be interpreted as having the same meaning as constituent elements in plural form.

Although explicitly stated otherwise, a range of allowable errors for a constituent element should be considered when interpreting the constituent element.

The terms first, second, and so on, although used to describe various constituent elements, do not impose any limitation on these terms. These terms are used to distinguish one constituent component from one or more other constituent components. Therefore, a first constituent element that will be described below may be named a second constituent element as long as this naming falls within the scope of the technical idea of the present disclosure.

Although explicitly stated otherwise, throughout the specification, the same reference numeral refers to the same constituent element.

Features of the various embodiments of the present disclosure may be integrated or combined severally or as a whole. It would be sufficiently understood by a person of ordinary skill that various conjunctive or driving operations are technically possible. The embodiments may be implemented independent of each other or may be implemented in conjunction with each other.

An inherent effect expected from technical features of the present disclosure, which is not specifically mentioned in the present specification of the present disclosure, is regarded as being incorporated in the present specification. The embodiments are provided to enable a person of ordinary skill in the art to practice the present disclosure without undue experimentation. For this reason, constituent elements of the present disclosure may be illustrated in a different non-proportional manner in the drawings, compared to their actual appearance. A detailed description of a constituent may be omitted when determined to make the nature and gist of the present disclosure unnecessarily obfuscated, or may be shortened.

In the present specification, light having a plurality of wavelengths may refer to light configured as one beam simultaneously having a plurality of different wavelengths. Additionally, this light may refer to light configured as a plurality of different beams, each corresponding to a wavelength, or may refer to light (wavelength-variable light) that varies in wavelength over time in one beam. Accordingly, light sources releasing light having a plurality of wavelengths may include multi-wavelength light sources, multi-array light sources, wavelength-variable light sources, and similar wavelengths. These light sources may also release light, having different wavelengths, simultaneously or at different times.

In the present specification, illumination light is light used for the purpose of brightly illuminating a sample, and refers to bright light having a wavelength range of visible light. Particularly, in several implementation examples of the present disclosure, illumination light may be white light, and, preferably, an illumination fixture that releases this illumination light may be realized using a white LED.

In the present specification, a control apparatus is an apparatus capable of controlling constituent elements of a multi-spectrum fluorescence imaging system according to the present disclosure. The control apparatus establishes a wired or wireless connection to a light-emitting apparatus and a light detection apparatus according to the present disclosure in a manner that electrically communicates them. The control apparatus may generate and supply a signal for controlling them. The control apparatus includes a processor and a memory, and examples of the control apparatus may include computers, mobile terminals, various electronic apparatuses with embedded programs, and similar apparatuses. In addition, the control apparatus may further include an image processing processor that receives an optical signal through the light detection apparatus according to an embodiment of the present disclosure and processes and outputs an image. Moreover, the control apparatus may also output the image, processed by the image processing processor, through a display device connected thereto. The control apparatus is not limited to the above-mentioned apparatuses, and various examples of the control apparatus include all apparatuses that are capable of generating and transmitting a signal for controlling the multi-spectrum fluorescence imaging system and of processing data received from the multi-spectrum fluorescence imaging system.

Embodiments of the present disclosure will be described in detail below with reference to the accompanying drawings in a manner that enables a person of ordinary skill in the art to which the present disclosure pertains to practice the present disclosure without undue experimentation. The present disclosure may be practiced in various different forms and is not limited to the embodiments described in the present specification. For definite description of the present disclosure, a constituent element not related to the description thereof is omitted from the drawings. Throughout the specification, the same or similar constituent elements are given the same reference numeral. The size and thickness of each of the constituent elements that are illustrated in the drawings are arbitrarily given for convenient description, and therefore, the present disclosure is not necessarily limited to this size and thickness.

The present disclosure will be described in detail below with the accompanying drawings.

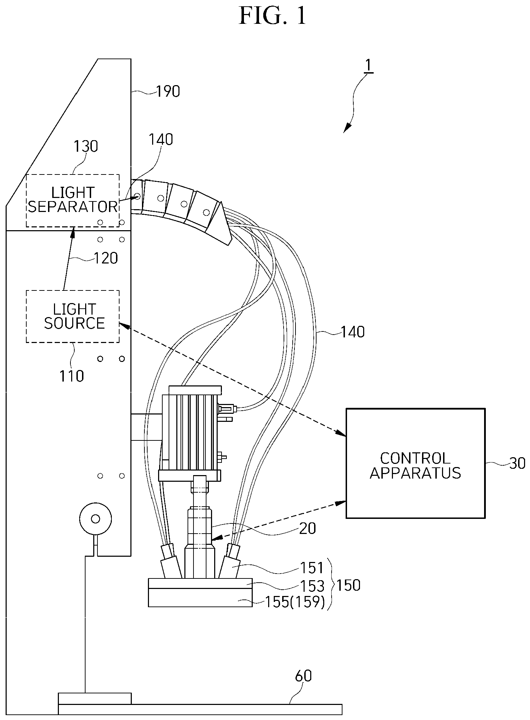

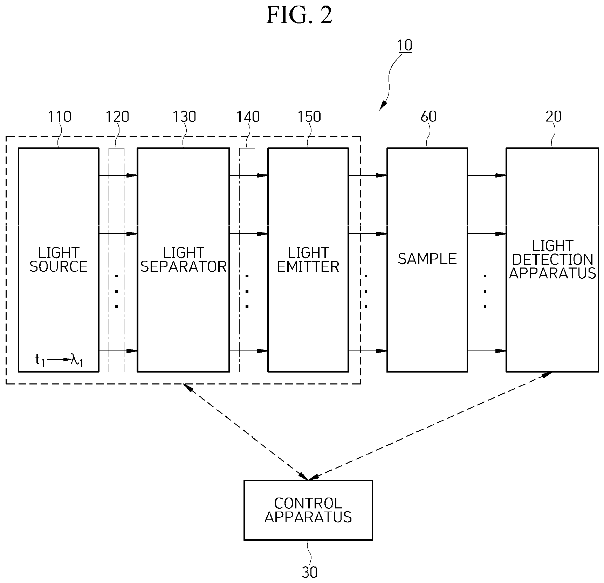

FIG. 1 is a view schematically illustrating a multi-spectrum fluorescence imaging system 1 according to a first embodiment of the present disclosure. FIG. 2 is a schematic block diagram illustrating an entire configuration of the multi-spectrum fluorescence imaging system 1 according to the first embodiment of the present disclosure.

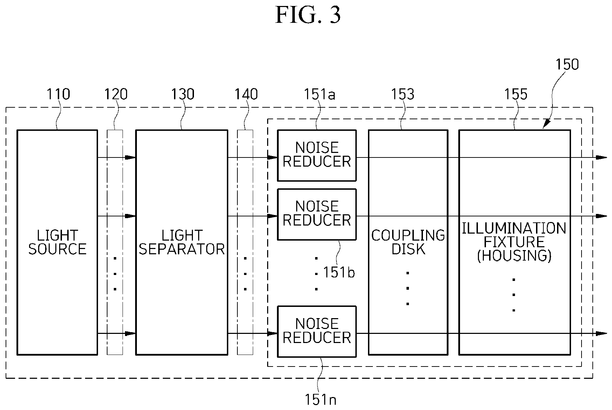

With reference to FIGS. 1 and 3, the multi-spectrum fluorescence imaging system 1 according to the first embodiment of the present disclosure includes a multi-spectrum light-emitting apparatus (hereinafter referred to as a "light-emitting apparatus") 10, a multi-spectrum light detection apparatus (hereinafter referred to as a "light detection apparatus") 20, and a control apparatus 30.

Specifically, the light-emitting apparatus 10 includes a light source 110, a light source multi-mode fiber 120, a light separator 130, a plurality of multi-mode fibers 140, and a light emitter 150.

The light source 110 is configured to release light having a plurality of wavelengths. Preferably, the light source 110 may release various types of lasers. For example, the light source 110 may refer to a laser using a semiconductor, a quantum dot laser, or a semiconductor optical amplifier light source.

The light source 110 may be configured to release light that varies in wavelength over time. Specifically, the light source 110 may be one of the following: a wavelength-variable light source, a multi-wavelength light source, or a multi-array light source. A specific relationship between a technique of releasing light according to a type of the light source 110 and the light source multi-mode fiber 120 will be described below with reference to FIG. 3.

The light source multi-mode fiber 120 transfers light, released from the light source 110, to the light separator 130. The light source multi-mode fiber 120 connects between the light source 110 and the light separator 130. That is, the light source multi-mode fiber 120 may be a fiber strand, as a multi-mode fiber, that connects the light source 110 and the light separator 130, and may also include as many individual fiber strands as the number of wavelengths of light to be released. The light source multi-mode fiber 120 may be configured with a plurality of fiber strands, and the appropriate number of fibers may be used according to the type of the light source 110. A specific relationship between the light source multi-mode fiber 120 and the light source 110 will be described below with reference to FIG. 3.

The light separator 130 separates light having a plurality of wavelengths, which is received through the light source multi-mode fiber 120, into a plurality of individual beams of light. Specifically, the light separator 130 combines light, transferred through the light source multi-mode fiber 120, into one flux. Then, the light separator 130 separates the flux into a plurality of individual beams of light, each having an individual wavelength, for transfer to the plurality of multi-mode fibers 140, respectively. In addition, the light separator 130 separates one flux, resulting from light combination, into a plurality of individual beams of light, each having predetermined individual power, for transfer to the plurality of multi-mode fibers 140, respectively.

The plurality of individual beams of light, resulting from the separation in the light separator 130, are transferred to the light-emitting apparatus 150 over the plurality of multi-mode fibers 140, respectively. Individual light having one individual wavelength may be transferred over each of the plurality of multi-mode fibers 140. Alternatively, individual light that has one power level, but is configured to have a plurality of wavelengths, may be transferred over each of the plurality of multi-mode fibers 140. A specific configuration of the light separator 130 in which the separation into the plurality of individual beams of light and the transfer to the plurality of multi-mode fibers 140 take place will be described below with reference to FIG. 3.

The light emitter 150 is connected to one end portion of each of the plurality of multi-mode fibers 140. The light emitter 150 reduces noise in individual light and emits the resulting individual light, along with illumination light, to the sample. In this manner, the light emitter 150 may emit the plurality of individual beams of light having different wavelengths to the sample, simultaneously or sequentially, and may also continuously emit illumination light while emitting the plurality of individual beams of light. The light emitter 150 includes a noise reducer 151, a coupling disk 153, and an illumination fixture 155. A specific configuration of the light emitter 150 will be described below with reference to FIGS. 3 to 6.

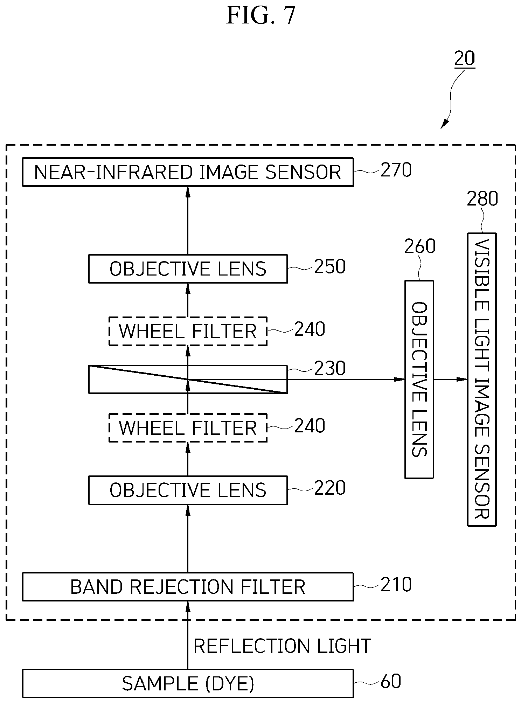

The light detection apparatus 20 detects a reflection light that results from light emitted from the light-emitting apparatus 10 toward a sample 60 being reflected off from the sample 60 and then being transferred to the light detection apparatus 20. The light detection apparatus 20 receives the reflection light through an objective lens arranged toward the sample 60. The light detection apparatus 20 that receives the reflection right may be coupled to the light emitter 150. A specific configuration of the light detection apparatus 20 will be described below with reference to FIG. 7.

The control apparatus 30 synchronizes the light-emitting apparatus 10 and the light detection apparatus 20 with each other. The control apparatus 30 may receive data on the time at which light is released and on a wavelength of the released light, from the light-emitting apparatus 10, and may receive data on the time at which detected light is received and on a wavelength of the detected light, from the light detection apparatus 20. The control apparatus 30 may obtain information on the detected light in a more precise manner by synchronizing the data on the time at which light is released by the light-emitting apparatus 10 and on the wavelength of the emitted light and the data on the time at which the light is detected by the light detection apparatus 20 and on the wavelength of the detected light. The control apparatus 30, as illustrated in FIG. 1, may be mounted within a housing 190 connected to the light-emitting apparatus 10 and the light detection apparatus 20 and may be connected to outside of the housing 190.

FIG. 3 is a schematic block diagram illustrating the multi-spectrum light-emitting apparatus according to the first embodiment of the present disclosure.

The light source multi-mode fiber 120 may be configured with one or more optical fibers. FIG. 3 illustrates that the light source multi-mode fiber 120 is configured with a plurality of optical fibers, but in an illustrative manner. The light source multi-mode fiber 120 may also be configured with one optical fiber. Accordingly, light having different wavelengths may be transferred from the light source 110 toward the light separator 130 over one light source multi-mode fiber 120 among several light source multi-mode fibers. Alternatively, light having different wavelengths may be separated and transferred to the light separator 130 over each of a plurality of light source multi-mode fibers 120.

The light source 110 may also release different lasers having different wavelengths, at different times over different light source multi-mode fibers 120, respectively (the multi-wavelength light source). The light source 110 may also release a laser having a desired wavelength, at different times over one light source multi-mode fiber 120 (the wavelength-variable light source). The light source 110 may also release lasers having different wavelengths, simultaneously over different light source multi-mode fibers 120, respectively (the multi-array light source). For example, in a case where the light source 110 is the multi-wavelength light source, the light source 110 may release a laser having a specific wavelength λ1, at an arbitrary time t1 over one light source multi-mode fiber 120a. Furthermore, the light source 100 may release a laser having another specific wavelength λ2, at another time t2 over another light source multi-mode fiber 120b. Furthermore, the light source 110 may release a laser having still another specific wavelength λ3, at still another time t3 over still another light source multi-mode fiber 120c. In addition, in a case where the light source 110 is the wavelength-variable light source, the light source 110 may release a laser having a specific wavelength λ1 during a time period from an arbitrary time t1 before another time t2 over one light source multi-mode fiber 120a. Furthermore, the light source 110 may release a laser having another specific wavelength λ2 during a time period from another arbitrary time t2 before still another time t3 over another light source multi-mode fiber 120b. Furthermore, the light source 110 may release a laser having still another specific wavelength λ3 during a time period from still another arbitrary time t3 before still another time t4 over still another light source multi-mode fiber 120c. In this manner, the number of the light source multi-mode fibers 120 in use may vary according to a type of the light source 110.

The plurality of multi-mode fibers 140 may be arranged in such a manner that they correspond to the light source multi-mode fibers 120, respectively. For example, the number of the plurality of multi-mode fibers 140 may be the same as the number of the light source multi-mode fiber 120.

In a case where the separation into a plurality of different beams of light takes place by the light separator 130, the plurality of different beams of light, resulting from the separation, are transferred to the light emitter 150 over different multi-mode fibers 140, respectively, according to their respective individual wavelength. In addition, in a case where, by the light separator 130, beams of light having a plurality of wavelengths are combined and the resulting light is separated into a plurality of individual beams of light having different individual power levels, the plurality of individual beams of light, resulting from the separation, are transferred to the light emitter 150 over different multi-mode fibers 140, respectively, according to their respective magnitudes of the individual power levels.

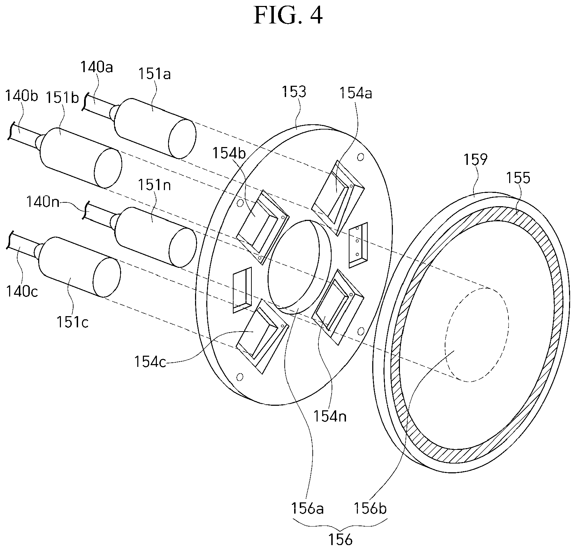

With reference to FIG. 3, the light emitter 150 includes a plurality of noise reducers 151, the coupling disk 153 (not illustrated), which fixes and supports the plurality of noise reducers 151, and the illumination fixture 155. The illumination fixture 155 releases illumination light, and, preferably, the illumination light may be white light. The light emitter 150 is connected to one end portion of each of the plurality of multi-mode fibers 140, and, more specifically, the noise reducer 151 may be connected to one end portion of each of the plurality of multi-mode fibers 140. In several implementation examples of the present disclosure, the noise reducer 151 may not be coupled to one end portion of each of the several multi-mode fibers 140. An individual beam of near-infrared light in a range of specific frequencies may be released over the multi-mode fiber 140 to which the noise reducer 151 is not coupled in this manner. This individual beam of near-infrared light may be used for the treatment purpose of killing cancer cells. A specific configuration of the light emitter 150 and various implementation examples thereof will be described below with reference to FIGS. 4 to 6.

The multi-spectrum light-emitting apparatus 10 included in the multi-spectrum fluorescence imaging system 1 according to the first embodiment of the present disclosure, along with a light source releasing light having a plurality of wavelengths, emits illumination light to the sample 60. Thus, individual fluorescent images that result from a plurality of wavelengths can be realized by identifying a multi-spectrum of light emitted to the sample 60. That is, the light-emitting apparatus 10 according to the first embodiment of the present disclosure may detect portions of the sample 60 that are marked with contrast mediums, in one go by releasing light having a plurality of different wavelengths. In addition, the light-emitting apparatus 10 according to the first embodiment of the present disclosure includes the noise reducer 151 and may eliminate speckle noise from beams of light that pass through the noise reducer 151 and are emitted to the sample 60. Moreover, the light-emitting apparatus 10 according to the first embodiment of the present disclosure may release an individual beam of light having a plurality of wavelengths, along with illumination light with which the sample 60 is illuminated. Thus, the light-emitting apparatus 10 may illuminate the sample 60 brightly and, at the same time, may acquire a visible light signal and fluorescent signals that correspond to the plurality of wavelengths, respectively.

FIG. 4 is an exploded perspective view illustrating the light emitter 150 according to the first embodiment of the present disclosure.

With reference to FIG. 4, the light emitter 150 includes the plurality of noise reducers 151, the coupling disk 153, and the illumination fixture 155.

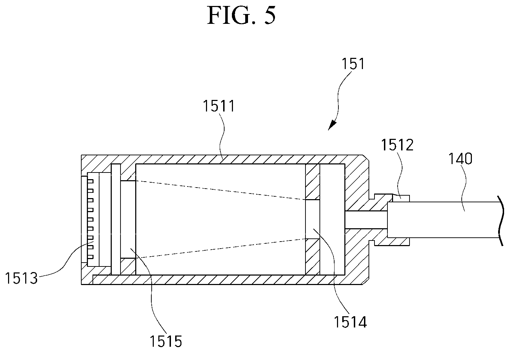

The plurality of noise reducers 151 are connected to the plurality of multi-mode fibers 140, respectively, and thus, reduce noise in an individual beam of light that is to be transferred over the plurality of multi-mode fibers 140, respectively. Specifically, the noise reducer 151 eliminates or reduces speckle noise included in a laser having a specific wavelength and specific power, which is an individual beam of light that is transferred over the connected multi-mode fiber 140. A specific configuration of the noise reducer 151 for eliminating this noise in the laser will be described below with reference to FIG. 5.

The coupling disk 153 includes a plurality of coupling openings, that is, coupling openings 154a, 154b, 154c, and so forth up to 154n and a lens opening 156a. The coupling disk 153 is configured in such a manner that the plurality of noise reducers 151 are arranged to be spaced a predetermined distance apart from each other. Specifically, the plurality of noise reducers 151, that is, noise reducers 151a, 151b, 151c, and so forth up to 151n, are coupled to the plurality of coupling openings, that is, the coupling openings 154a, 154a, 154b, 154c, and so forth up to 154n, respectively, in the coupling disk 153 in such a manner as to be arranged to be spaced a predetermined distance apart from each other. FIG. 4 illustrates four coupling openings 154, but the present invention is not limited thereto. In various implementation examples of the present disclosure, the coupling openings 154 may be configured in such a manner that the number of the coupling openings 154 corresponds to the number of the noise reducers 151. In addition, the present disclosure is not limited to four multi-mode fibers 140 and four noise reducers 151 that are illustrated in FIG. 4. In various implementation examples of the present disclosure, an arbitrary number of multi-mode fibers 140 and an arbitrary number of noise reducers 151 may be configured.

The illumination fixture 155 is coupled to one surface of the coupling disk 153 and is arranged to surround the vicinity of the plurality of noise reducers 151. Preferably, the illumination fixture 155 may be mounted in an illumination housing 159 that comes into contact with one surface of the coupling disk 153 or is coupled toward one surface of the coupling disk 153. Alternatively, the illumination fixture 155 may be mounted on another disk.

The illumination housing 159 may fix and support the illumination fixture 155. In addition, the illumination housing 159 may be coupled to one surface of the coupling disk 153 or may be coupled to the coupling disk 153 in such a manner as to surround the coupling disk 153. Openings may be formed in a portion of the illumination housing 159, this position being positioned inward from an inner circumferential surface of the illumination fixture 155. The plurality of noise reducers 151 pass through the openings, respectively, in such a manner as to protrude from the illumination housing 159. Particularly, the entire portion of the illumination housing 159, the entire portion being positioned inward from an inner circumferential surface of the illumination fixture 155, may be made empty. Alternatively, openings may be formed in the entire portion of the illumination housing 159, the entire portion being positioned inward from an inner circumferential surface of the illumination fixture 155, in a manner that they correspond to the plurality of noise reducers 151 or the coupling openings 154, respectively. Alternatively, an opening may be formed in the entire portion of the illumination housing 159, the entire portion being positioned inward from an inner circumferential surface of the illumination fixture 155, in a manner that corresponds to the lens opening 156a.

In addition, a lens opening 156b may be arranged at a position on the illumination housing 159 to which the illumination fixture 155 is coupled and fixed, this position corresponding to a position of the lens opening 156a in the coupling disk 153. The lens openings 156a and 156b may collect beams of light that is emitted to the sample 60 by the light emitter 150 and is reflected off from the sample 60 and may transfer the collected beams of light to the light detection apparatus 20.

The light emitter 150 according to the first embodiment of the present disclosure includes the noise reducers 151, of which the number is the same as the number of the multi-mode fibers 140. Each of the noise reducers 151 is individually connected to one end portion of each of the multi-mode fiber 140. In this manner, the noise reducers 151 are connected to the multi-mode fibers 140, respectively. Thus, noise in a beam of light that is released through each of the multi-mode fiber 140 can be efficiently eliminated or reduced. Particularly, in a case where the beam of light is a laser, serious speckle noise occurs due to interference between the beams of light, and multi-modal noise can occur due to the use of a multi-mode fiber. The connection of the noise reducers 151 to the multi-mode fibers 140, respectively, can efficiently eliminate or reduce at one time various types of noise, such as the speckle noise and the multi-modal noise.

FIG. 5 is a cross-sectional view illustrating the noise reducer 151 according to the first embodiment of the present disclosure.

With reference to FIG. 5, the noise reducer 151 includes a cylindrical housing 1511, an optical fiber connection portion 1512, a glass pane 1513, an aspherical lens 1514, and a homogenizing lens or diffusing lens 1515. Specifically, the noise reducer 151 includes: a cylindrical housing 1511; an optical fiber connection portion 1512 connected to the multi-mode fiber 140 at one end portion of the cylindrical housing 1511; a glass pane 1513 installed at the other end portion of the cylindrical housing 1511; an aspherical lens 1514 arranged within the cylindrical housing 1511 in a manner that is adjacent to optical fiber connection portion 1512; and a homogenizing lens or diffusing lens 1515 arranged within the cylindrical housing 1511 in a manner that is adjacent to the glass pane 1513.

The individual beam of light transferred through the multi-mode fiber 140 passes through the optical fiber connection portion 1512 and reaches the aspherical lens 1514. An observation field of view (FoV) of the individual beam of light passing through the aspherical lens 1514 is adjusted, and the resulting individual beam of light is transferred to the homogenizing lens or diffusing lens 1515. The individual beam of light may have uniform characteristics through the homogenizing lens or diffusing lens 1515, and the individual beam of light with uniform characteristics may pass through the glass pane 1513 and may be emitted to the sample 60. The observation field of view may be adjusted according to the size, the thickness, the arrangement position, or the like of each of the glass pane 1513, the aspherical lens 1514, and the homogenizing lens or diffusing lens 1515 within the noise reducer 151. Preferably, the aspherical lens 1514 has a smaller diameter than the homogenizing lens or diffusing lens 1515, and the homogenizing lens or diffusing lens 1515 has a smaller diameter than the glass pane 1513. Particularly, the observation field of view may be adjusted by adjusting the diameter, the thickness, the arrangement position, or the like of the aspherical lens 1514.

As described above, the noise reducers 151 according to the first embodiment of the present disclosure can eliminate or reduce various types of noise, such as the speckle noise and the multi-modal noise by the multi-mode fibers 140, respectively. The observation field of view may be adjusted more finely and easily by changing the characteristics, the structure, and the position, and the like of each of the lenses that constitute the noise reducer 151.

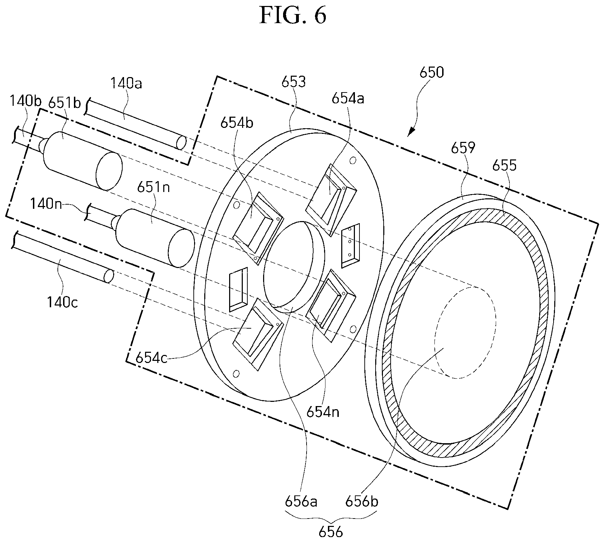

FIG. 6 is an exploded perspective view illustrating a light emitter 650 according to a second embodiment of the present disclosure. The light emitter 650 in FIG. 6 has substantially the same configuration as the light emitter 150 illustrated in FIG. 4 in that there is a difference in the number of noise reducers, and thus descriptions of substantially the same constituent elements are omitted.

FIG. 6 illustrates a case where the number of the noise reducers 651 is smaller than the number of the coupling openings 654. In this case, the number of the noise reducers 651 is smaller than the number of the multi-mode fibers 140. FIG. 6 illustrates two noise reducers 651b and 651n, four multi-mode fibers 140a, 140b, 140c, and 140n, and four coupling openings 654a, 654b, 654c, and 654n.

In this case, the noise reducers 651b and 651n may be selectively coupled to the coupling openings 654a, 654b, 654c, and 654n in an arbitrary manner or at efficient positions. For example, the second noise reducer 651b may be coupled to the second coupling opening 654b, and the fourth noise reducer 651n may be coupled to the fourth coupling opening 654n. In addition, the first multi-mode fiber 140a and the third multi-mode fiber 140c that are not connected to the noise reducer 651b and 651n may be coupled in an arbitrary way, to the first coupling opening 654a and the third coupling opening 654c, respectively, that are the remaining coupling openings.

Particularly, the multi-mode fibers 140a and 140c to which the noise reducers 651b and 651n are not coupled may release light having a specific wavelength to the sample 60. Specifically, the multi-mode fibers 140a and 140c to which the noise reducers 651b and 651n are not coupled may release a ray of light having a wavelength of between 200 nm to 450 nm, a ray of light having a wavelength of between 510 nm to 550 nm, a beam of near-infrared light having a wavelength of between 790 nm to 980 nm, or a beam of near-infrared light having a wavelength of between 1044 nm to 1084 nm. Preferably, the multi-mode fibers 140a and 140c to which the noise reducers 651b and 651n are not coupled may release a beam of near-infrared light having a wavelength of 808 nm to the sample 60. With this configuration, the multi-mode fibers 140b and 140n to which the noise reducers 651b and 651n are coupled may release an individual beam of light having a specific wavelength from which various types of noise are eliminated and, at the same time, the multi-mode fibers 140b and 140n to which the noise reducers 651b and 651n are not coupled may directly emit an individual beam of light having a specific wavelength to the sample 60. Accordingly, in a case where the sample 60 is a cancer cell, a beam of near-infrared light, capable of detecting various cancer cells marked with contrast mediums in one go, is emitted toward the cancer cells over the multi-mode fibers 140b and 140n to which the noise reducers 651b and 651n are coupled. At the same time, a laser having a specific wavelength, which kills cancer cells, is emitted over the multi-mode fibers 140a and 140n to which the noise reducers 651b and 651 are not coupled. In this manner, the cancer cells are identified and immediately treated directly and rapidly.

Two multi-mode fibers 140b and 140n, to which the noise reducers 651b and 651n are coupled, and two multi-mode fibers 140a and 140c, to which the noise reducers 651b and 651n are not coupled, are described with reference to FIG. 6. However, the number of the multi-mode fibers to which the noise reducers are coupled, and the number of the multi-mode fibers to which the noise reducers are not coupled are not limited to two that are illustrated in FIG. 6 and may be changed in various implementation examples. That is, the number of the multi-mode fibers 140b and 140n to which the noise reducers 651b and 651n are coupled may be larger or smaller than the number of the number of the multi-mode fibers 140a and 140c to which the noise reducers 651b and 651n are not coupled.

FIG. 7 is a schematic block diagram illustrating the multi-spectrum light detection apparatus 20 according to the first embodiment of the present disclosure.

With reference to FIGS. 1, 2, and 7, the light released from the light-emitting apparatus 10 reaches the sample 60, and the optical signal that reaches the light detection apparatus 20 is separated into a visible light signal reflected off from the sample 60 and a near-infrared signal released from the sample 60. The light detection apparatus 20 may identify and detect the visible light signal and the near-infrared signal, which result from the separation. The near-infrared signal here is a near-infrared fluorescent signal. The light detection apparatus 20 includes a band rejection filter (BRF) 210, a plurality of objective lenses, that is, objective lenses 220, 250, and 260, a wavelength separator 230, a near-infrared image sensor 270, and a visible light image sensor 280. In addition, the light detection apparatus 20 may further include a wheel filter 240.

With reference to FIG. 7, the band rejection filter 210 is arranged between the sample 60 and the wavelength separator 230. In addition, the band rejection filter 210 may be arranged closer to the sample 60 than the objective lens 220. The band rejection filter 210 may eliminate an exciting light component from a reflection light, reflecting from the light being reflected off from the sample 60, by eliminating a wavelength of exciting light from the reflection light. Accordingly, the light detection apparatus 20 may receive near-infrared signals in accordance with beams of light corresponding to specific wavelengths, which are released from the light source 110, and a visible light signal in accordance with illumination light, without noise due to the exciting light. The near-infrared signal here consequently corresponds to a fluorescent signal, and therefore, the near-infrared signal other than the visible light signal may be considered to have the same meaning as the fluorescent signal.

The optical signal from which the exciting light component is eliminated by the band rejection filter 210 passes through the objective lens 220 adjacent to the band rejection filter 210 and reaches the wavelength separator 230. The wavelength separator 230 is configured to separate the optical signal on the basis of a desired wavelength, and examples thereof may include a dichroic mirror and a beam splitter.

The near-infrared fluorescent signal includes a component of a laser that corresponds to a specific wavelength, released from the light source 110, and the visible light signal includes a component that results from illumination light being reflected off from the sample 60. Accordingly, the wavelength separator 230 separates the optical signal into a visible light signal reflected off from the sample 60, and a near-infrared fluorescent signal released from the sample 60. The near-infrared fluorescent signal, resulting from the separation by the wavelength separator 230, is sent to the near-infrared image sensor 270, and the visible light signal, resulting from the separation by the wavelength separator 230, is sent to the visible light image sensor 280.

In another implementation example of the present disclosure, the light detection apparatus 20 may further include the wheel filter 240. The wheel filter 240 is a filter in the shape of a wheel, and may include a plurality of band pass filters (BPFs). Preferably, the plurality of band pass filters, which constitute the wheel filter 240, may be arranged to be spaced a predetermined distance apart from each other along a wheel-shaped circumference. Each of the band pass filters may allow only a signal having a specific wavelength component, among fluorescent signals released from the sample 60, to pass through.

For example, the wheel filer 240 may include n band pass filters, and each of the n band pass filters may allow near-infrared light in a range of n different specific wavelengths to pass through. Accordingly, even though light incident on the wheel filter 240 has n or more wavelengths, an optical signal passing through the wheel filter 240 may include only a signal component in a range of specific wavelengths.

FIG. 7 illustrates that the wheel filter 240 is arranged between the wavelength separator 230 and the objective lens 250. However, this arrangement is only one of various implementation examples. The wheel filter 240 may be arranged at a position where a wavelength is selectively allowed to pass through. Specifically, the wheel filter 240 may be arranged between the wavelength separator 230 and the near-infrared image sensor 270 or between the wavelength separator 230 and the band rejection filter 210. More specifically, the wheel filter 240, as illustrated in FIG. 7, may also be arranged between the wavelength separator 230 and the objective lens 250 arranged before the near-infrared image sensor 270 and may also be arranged between the objective lens 220 arranged after the band rejection filter 210 and the wavelength separator 230.

The near-infrared fluorescent signal, resulting from the separation by the wavelength separator 230, passes through the objective lens 250 and reaches the near-infrared image sensor 270. The near-infrared image sensor 270 may detect a near-infrared signal having a plurality of wavelengths. Particularly, the near-infrared image sensor 270 may separate near-infrared fluorescent signals having a plurality of wavelengths into near-infrared fluorescent signals, each per wavelength and may detect the resulting near-infrared fluorescent signals. Accordingly, the near-infrared image sensor 270 may transfer the detected near-infrared fluorescent signals, each per wavelength, to the control apparatus 30 or the like and may output near-infrared images distinguished per wavelength.

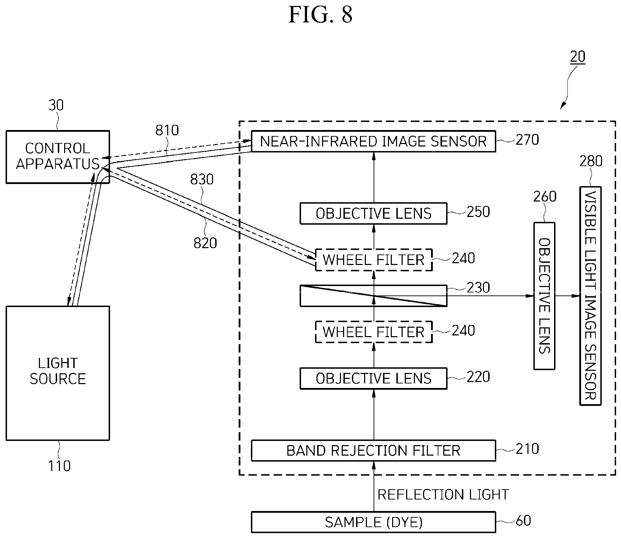

Specifically, the near-infrared image sensor 270 may receive data on the time at which light is released from the light source 110 and on a wavelength of the light and may be synchronized with the data, thereby detecting a near-infrared fluorescent signal. In addition, the near-infrared image sensor 270 may also be synchronized with the wheel filter 240. The wheel filter 240 may also be synchronized with the light source 110. A specific technique in which the near-infrared image sensor 270 is synchronized with the light source 110 and the wheel filter 240 will be described below with reference to FIG. 8.

The visible light signal, resulting from the separation by the wavelength separator 230, passes through the objective lens 260 and reaches the visible light image sensor 280. The visible light image sensor 280 may detect this visible light signal. Accordingly, the visible light image sensor 280 may transfer the detected visible light signal to the control apparatus 30 or the like in such a manner as to output a visible light image.

As described below, the wavelength separator 230 of the light detection apparatus 20 according to the first embodiment of the present disclosure may perform separation into near-infrared fluorescent signals, which correspond to a plurality of wavelengths, respectively, and a visible light signal. The light detection apparatus 20 may detect the signals, resulting from the separation. Consequently, the near-infrared fluorescent signals, corresponding to the plurality of wavelengths, correspond to fluorescent signals corresponding to the plurality of wavelengths. Therefore, the light detection apparatus 20 may perform separation into the fluorescent signals, corresponding to the plurality of wavelengths, respectively, and the visible light signal, and may detect the signals, resulting from the separation. That is, in one multi-spectrum fluorescence imaging system 1 according to the first embodiment of the present disclosure, the light detection apparatus 20 may simultaneously receive and detect near-infrared fluorescent signals having a plurality of wavelengths and a visible light signal and may perform separation into a plurality of near-infrared fluorescent signals and the visible light signal on the basis of a wavelength. The plurality of near-infrared fluorescent signals and the visible light signal, which result from this separation, can be displayed in a state of being distinguished using predetermined specific colors. As a result, the multi-spectrum fluorescence imaging system 1 according to the first embodiment of the present disclosure, which includes the light detection apparatus 20, may release light having a plurality of wavelengths the sample 60 in one go, using the multi-wavelength light source, the multi-array light source, or the wavelength-variable light source. Accordingly, a visible light signal and a near-infrared fluorescent signal per wavelength can be separated in one go from an optical signal made up of a plurality of wavelengths, which is reflected and radiated from the sample 60. These signals can be output in different colors in a state of being distinguished from each other.

FIG. 8 is a block diagram that is referenced to a method of synchronizing the multi-spectrum light-emitting apparatus 10 and the multi-spectrum light detection apparatus 20 with each other in the multi-spectrum fluorescence imaging system 1 according to the first embodiment of the present disclosure. FIG. 8 illustrates substantially the same constituent elements as those in FIG. 7 that is referenced to describe the method of the synchronization. The same respective descriptions of the constituent elements are omitted.

With reference to FIG. 8, information on light that is released and transferred between the light source 110 and the near-infrared image sensor 270 is synchronized (810), information on light that is released and transferred between the light source 110 and the wheel filter 240 is synchronized, (820) and information on light that is released and transferred between the wheel filter 240 and the near-infrared image sensor 270 (830).

This synchronization process may be performed through the control apparatus 30 connected to the light-emitting apparatus 10 and the light detection apparatus 20. Specifically, the control apparatus 30 may synchronize a time between the light source 110 and the near-infrared image sensor 270 and a wavelength of light, which corresponds to that time (810), may synchronize the time between the light source 110 and the wheel filter 240 and a wavelength of light, which corresponds to that time (820), and may synchronize a time between the wheel filter 240 and the near-infrared image sensor 270 and a wavelength of light, which corresponds to that time (830). More specifically, the control apparatus 30 may receive or store information on a wavelength of light released from the light source 110 and on the time at which the light with such a wavelength is released, and may transfer the information on the wavelength of the released light and on the time at which the light is released, to the near-infrared image sensor 270 and the wheel filter 240. Accordingly, the near-infrared image sensor 270 and the wheel filter 240 may receive information on when light having a specific wavelength is released from a light source. Moreover, the wheel filter 240 and the near-infrared image sensor 270 may detect a fluorescent signal having a specific wavelength, from among fluorescent signals released from the sample 60, may detect the fluorescent signal having the specific wavelength in a more precise manner by synchronizing the time at which the fluorescent signal having the specific wavelength is released, and may identify the fluorescent signal having the specific wavelength, from fluorescent signals having different wavelengths.

The multi-spectrum fluorescence imaging system 1 according to the first embodiment of the present disclosure may synchronize information on the time at which light is released from the light-emitting apparatus 10 and on a wavelength of the released light and information on the time at which the light is received in the light detection apparatus 20 and on a wavelength of the received light. Accordingly, in the multi-spectrum fluorescence imaging system 1, in a more precise manner on the basis of the synchronized information on the light, the light detection apparatus 20 may perform separation of individual fluorescent signals for a plurality of wavelengths, respectively, and may identify and detect the resulting individual fluorescent signals. Accordingly, although light having a plurality of wavelengths is released at one time or at different times, the use of the multi-spectrum fluorescence imaging system 1 enables the individual fluorescent signals for the plurality of wavelengths, respectively, to be detected and identified in a more precise manner. Thus, various types of contrast mediums can be used in one step. Moreover, various fluorescent signals having different wavelengths due to various type contrast mediums can be distinguished at one time, and thus lesions in various cell regions and states of cells can be checked in a single step.

In the present specification, each block may indicate one portion of a module, a segment, or a code that includes one or more executable instructions for performing a specific logical function(s). In addition, it is noted that, in several implementation examples, functions mentioned in blocks are performed regardless of the order thereof. For example, in practice, two blocks that are successively illustrated may be performed substantially simultaneously, and the two blocks or steps may be performed in the reverse order according to the corresponding function, depending on the case.

The embodiments of the present disclosure are described in more detail above with reference to the accompanying drawings. However, the present disclosure is not necessarily limited to the embodiments. Various modifications to the embodiments are possibly implemented within the scope that does not depart from the technical idea of the present invention. Therefore, the embodiments of the present disclosure that are disclosed in the present specification are for describing, rather than limiting, the technical idea of the present disclosure and do not impose any limitation on the scope of the technical idea of the present disclosure. Therefore, in every aspect, the embodiments described above should be understood as being exemplary and non-restrictive. Accordingly, the scope of protection of the present disclosure should be defined by the following claims. All technical ideas that fall within the scope equivalent thereto should be interpreted to be included within the scope of the claims of the present disclosure.

## Claims

1. A multi-spectrum light-emitting apparatus comprising:
a light source releasing light having a plurality of wavelengths;
a light separator separating the light having the plurality of wavelengths into a plurality of individual beams of light having individual wavelengths, or into a plurality of individual beams of light having predetermined power levels;
a plurality of multi-mode fibers, the plurality of individual beams of light, resulting from the separation by the light separator, being transferred over the plurality of multi-mode fibers, respectively; and
light emitters connected to respective end portions of the plurality of multi-mode fibers, thereby reducing noise in the plurality of individual beams of light and emitting the resulting plurality of individual beams of light, along with illumination light, to a sample.

2. The multi-spectrum light-emitting apparatus of claim 1, wherein the light source is a wavelength-variable light source, a multi-wavelength light source, or a multi-array light source, and releases light that varies in wavelength over time.

3. The multi-spectrum light-emitting apparatus of claim 1, further comprising:
a light source multi-mode fiber connecting between the light source and the light separator.

4. The multi-spectrum light-emitting apparatus of claim 1, wherein the light emitter comprises:
a plurality of noise reducers;
a coupling disk configured in such a manner that the plurality of noise reducers are arranged to be spaced a predetermined distance apart from each other; and
an illumination fixture coupled to one surface of the coupling disk and arranged to surround the vicinity of the plurality of noise reducers, and
wherein the illumination fixture releases the illumination light, and the illumination light is white light.

5. The multi-spectrum light-emitting apparatus of claim 4, wherein the number of the plurality of noise reducers is smaller than the number of the plurality of multi-mode fibers, and the multi-mode fiber to which the noise reducer is not coupled releases a ray of light having a wavelength of between 200 nm to 450 nm, a ray of light having a wavelength of between 510 nm to 550 nm, a beam of near-infrared light having a wavelength of between 790 nm to 980 nm, or a beam of near-infrared light having a wavelength of between 1044 nm to 1084 nm.

6. The multi-spectrum light-emitting apparatus of claim 4, wherein the plurality of noise reducers are connected to the plurality of multi-mode fibers, respectively, thereby reducing speckle noise and multi-modal noise in the plurality of individual beams of light transferred over the plurality of multi-mode fibers, respectively.

7. The multi-spectrum light-emitting apparatus of claim 4, wherein each of the plurality of noise reducers comprises:
a cylindrical housing;
an optical fiber connection portion connected to the multi-mode fiber at one end portion of the cylindrical housing;
a glass pane installed at the other end portion of the cylindrical housing;
an aspherical lens arranged adjacent to the optical fiber connection portion within the cylindrical housing; and
a homogenizing lens or diffusing lens arranged adjacent to the glass pane within the cylindrical housing.

8. A multi-spectrum fluorescence imaging system comprising:
the multi-spectrum light-emitting apparatus of claim 1;
a multi-spectrum light detection apparatus detecting a near-infrared fluorescent signal that is emitted from the multi-spectrum light-emitting apparatus to the sample and is released from the sample; and
a control apparatus synchronizing the multi-spectrum light-emitting apparatus and the multi-spectrum light detection apparatus with each other.

9. The multi-spectrum fluorescence imaging system of claim 8, wherein the multi-spectrum light detection apparatus comprises:
a wavelength separator separating an optical signal reaching the multi-spectrum light detection apparatus into a visible light signal reflected off from the sample and the near-infrared fluorescent signal released from the sample;
a band rejection filter arranged between the sample and the wavelength separator; a near-infrared image sensor detecting the near-infrared fluorescent signal; and
a visible light image sensor detecting the visible light signal.

10. The multi-spectrum fluorescence imaging system of claim 9, further comprising:
a wheel filter arranged between the wavelength separator and the near-infrared image sensor or between the wavelength separator and the band rejection filter.

11. The multi-spectrum fluorescence imaging system of claim 10, wherein the control apparatus synchronizes a time between the light source and the near-infrared image sensor and a wavelength of light, which corresponds to the time, synchronizes a time between the light source and the wheel filter and a wavelength of light, which corresponds to the time, and synchronizes a time between the wheel filter and the near-infrared image sensor and a wavelength of light, which corresponds to the time.
